Europäisches Patentamt

(19) European Patent Office

Office européen des brevets

(11) **EP 0 661 091 B1**

(12) **EUROPEAN PATENT SPECIFICATION**

(45) Date of publication and mention
of the grant of the patent:
**08.10.1997 Bulletin 1997/41**

(51) Int. Cl.⁶: **B01J 2/04**, C09D 5/03,
A61K 9/14

(21) Application number: **94119470.6**

(22) Date of filing: **09.12.1994**

(54) **Process for the production of powders with controlled particle sizes and powdery product so obtained**

Verfahren zum Herstellen von Pulvern mit kontrollierter Teilchengrösse und so erhaltene Pulver

Procédé pour produire des poudres ayant une grandeur de particule controlée et poudres obtenues

(84) Designated Contracting States:
**BE CH DE FR GB LI NL**

(30) Priority: **30.12.1993 IT MI932763**

(43) Date of publication of application:
**05.07.1995 Bulletin 1995/27**

(73) Proprietor: **OTEFAL S.p.A.**
**24050 Grassobbio (BG) (IT)**

(72) Inventors:
• **Mura, Gavino,**
**c/o Otefal S.r.l.**
**I-24050 Grassobbio (BG) (IT)**

• **Pozzoli, Silvio,**
**c/o Otefal S.r.l.**
**I-24050 Grassobbio (BG) (IT)**

(74) Representative: **Trupiano, Roberto**
**BREVETTI EUROPA S.r.l.**
**Piazza Bernini, 6**
**20133 Milano (MI) (IT)**

(56) References cited:
**WO-A-90/03782**

• **PATENT ABSTRACTS OF JAPAN vol. 13, no. 457 (C-644) 16 October 1989 & JP-A-01 176 437 (ONO PHARMACEUT CO LTD;)**

**Description**

This invention relates to a process for the production of powdery materials having a controlled granule size, especially suitable for materials constituted by mixes of several products; it also relates to the powdery materials or powders having controlled granule sizes obtained through said process.

Many intermediate and end industrial products for a wide range of applications are constituted by powders of one only substance or a mix of substances; for most of them, the granulometric characteristics are of great importance.

For instance, there are some cases in which a rather marked fineness of granule sizes and/or a dispersion of same as limited as possible is of the essential; or the granules may be required to have a shape as spherical as possible.

At present, the processes of fabrication of powders constituted by an intimate mix of several substances comprise typically the following stages:

a) mechanical mixing of components;
b) intimate homogenization of the mix brought to a pasty state and extrusion of same by means of a special precision equipment (mixer-extruder), to obtain the solid mix in the form of thin layers or other easily crumbling forms;
c) crushing of the extruded materials into pieces whose max. size depends on the characteristics of the grinder utilized in the following stage;
d) pulverization of the fragments by grinding, either normal or cryogenic for thermosensitive products, with various types of grinders; two or more passages are very often necessary;
e) screening, to remove the fraction of particles having too big or too small sizes.

The stages are limited to the above stages c, d and e, when one-substance powders are to be obtained, except for those which are obtained by crystallization by solution in a solvent; anyhow also these powders need often being ground.

The production of powders is therefore rather complicated and requires expensive equipments and also the work of several people; besides, the results are not always satisfactory for the users.

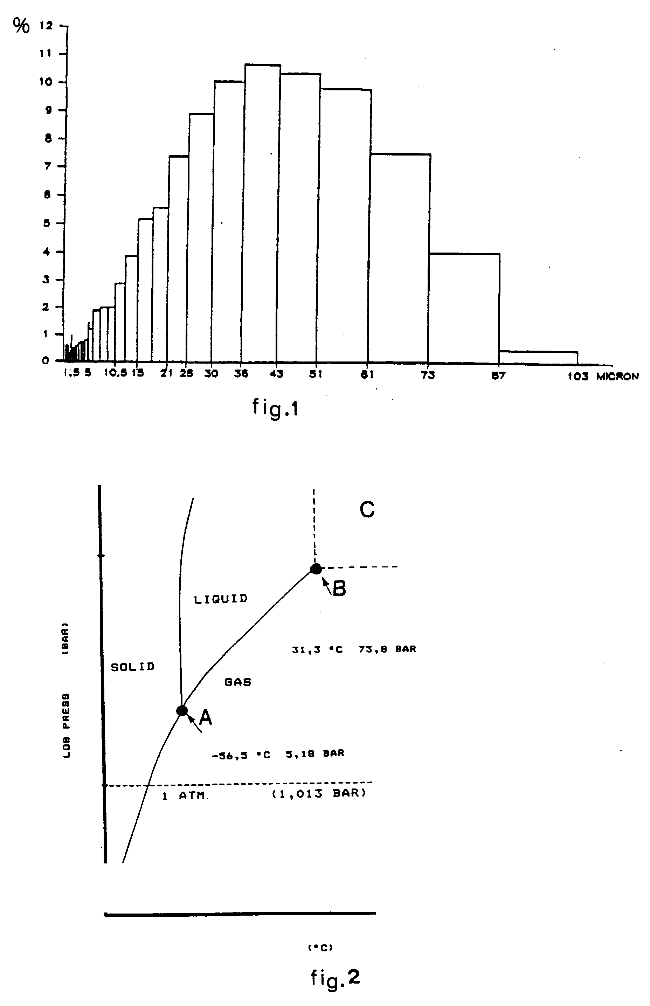

What one obtains is a distribution of granule sizes of the type of the one illustrated on Fig. 1, which refers to a powdery paint screened only to remove the coarsest particles, as often happens.

TABLE 1

| Weight percent under the upper limit | Interval $\mu$ | | Weight percent in the interval |
|---|---|---|---|
| 0,6 | 0,5 | 0,9 | 0,6 |

| | | | |
|---|---|---|---|
| 0,9 | 0,9 | 1,1 | 0,3 |
| 1,1 | 1,1 | 1,3 | 0,3 |
| 1,4 | 1,3 | 1,5 | 0,3 |
| 1,6 | 1,5 | 1,8 | 0,4 |
| 2,2 | 1,8 | 2,2 | 0,5 |
| 2,7 | 2,2 | 2,6 | 0,5 |
| 3,3 | 2,6 | 3,1 | 0,6 |
| 4,0 | 3,1 | 3,7 | 0,7 |
| 4,6 | 3,7 | 4,3 | 0,7 |
| 5,5 | 4,3 | 5,0 | 0,8 |
| 6,7 | 5,0 | 6,0 | 1,2 |
| 8,6 | 6,0 | 7,5 | 1,9 |
| 10,6 | 7,5 | 9,0 | 2,0 |
| 12,5 | 9,0 | 10,5 | 2,0 |
| 15,5 | 10,5 | 12,5 | 2,9 |
| 19,4 | 12,5 | 15,0 | 3,9 |
| 24,7 | 15,0 | 18,0 | 5,2 |
| 30,3 | 18,0 | 21,0 | 6,6 |
| 37,7 | 21,0 | 25,0 | 7,4 |
| 46,6 | 25,0 | 30,0 | 8,9 |
| 56,6 | 30,0 | 36,0 | 10,1 |
| 67,5 | 36,0 | 43,0 | 10,7 |
| 77,9 | 43,0 | 51,0 | 10,4 |
| 87,8 | 51,0 | 61,0 | 9,9 |
| 95,4 | 61,0 | 73,0 | 7,6 |
| 99,5 | 73,0 | 87,0 | 4,1 |
| 100,0 | 87,0 | 103,0 | 0,5 |

| 100,0 | 103,0 | 123,0 | 0,0 |
| 100,0 | 123,0 | 147,0 | 0,0 |
| 100,0 | 147,0 | 175,0 | 0,0 |

As one can see, the dispersion of granule sizes is very high, which involves the presence of rather high fractions of too coarse or too thin particles.

The mean size, understood as the size above which one finds 50% of the total weight, is about 32 $\mu$, as can be inferred from Table 1; considering the shape of the curve, as the fraction above 103 $\mu$ has been removed and the object of the producer is to achieve a mean size of about 40 $\mu$, 41 $\mu$ may be considered as a real approximate mean; while particles whose sizes are comprised between 21 and 60 $\mu$, i.e. in the interval around the mean of about $\pm$ 50%, are only 57.5% in weight against 30.3% of particles of smaller size and 12.2% of coarser particles, once the fraction in excess of 100 $\mu$ has been removed.

On the contrary, it would be suitable to obtain for the particles of this product a rather different size distribution, shifted towards middle-low sizes comprised between 5 and 20 $\mu$ and much less dispersed, with at least 80% of the material comprised within a max. interval of $\pm$ 20-30% compared to the mean and with negligible percents of particles under 4 $\mu$, which cannot be obtained with traditional equipments, for reason of both technical and economic nature.

Apart from the limitations and drawbacks which concern users, there are oher ones which concern the fabrication of the powders with the mechanical systems listed above.

Suffice to mention the difficulties and costs one has to bear to clean the machines, expecially extruders and grinders, very complicated and delicate equipments that generally need being dry-cleaned for environmental reasons, using solvents only for finishing-off the operation.

Also the control and maintenance of equipments is very expensive, both for the remarkable work and for the high cost of the spare parts of many precision parts subject to wear.

One should also consider the inelasticity of the production cycles, due to the fact that the dimensions and performances of the apparatuses depend on the production lots.

Besides, the costs of the equipments necessary to obtain thin particles and the associated maintenance costs increase remarkably when the sizes required are smaller.

There are also limitations concerning powders which have low melting or softening temperatures or which may alterate under the activity of heat.

For these substances it is even more difficult to obtain averagely finer particles or to limit their size dispersion, as also the mechanical system fittest to this aim, such as for instance high speed impact crushing, tends to generate comparatively high temperatures; even when one can utilize systems which do not involve heating, such as cryogenic grinding, other drawbacks appear: apart from the increase in costs for the equipment and the consumption of carbon dioxide and liquid nitrogen, there are time and production power losses due to the necessity of cleaning and replacing more often the parts of the machine, and also rather frequent drawbacks, such as the higher frequency of humidification of powders which in many cases cannot be tolerated.

Therefore, only the possibility of operating a posteriori would remain, i.e. by suitably screening the product obtained.

This technique (which obviously includes the recovery of scraps), even though it is adopted by many producers to remove the coarser particles and only by very few producers to remove the smaller ones, does not suffice to obtain an optimum product; besides, especially as concerns smaller particles, its cost is rather high.

First of all, the systems of separation through ventilation are increasingly less utilizable as the mean particle size decreases. On the other hand, the more one reduces the nominal opening of screens, the more screenings become expensive and delicate and also slower (taking also into account their lower capacity).

Besides, while for the recycle of coarse materials one can take into consideration their reintroduction in the grinding stage without too many drawbacks, thinner materials should be reintroduced in the extrusion stage: considering how much material one should reject, this would involve the need of increasing the production power of the machines, with financial and operating cost increases; besides, the addition of high amounts of fine powders in the extruder feedbox may also give rise to drawbacks ranging from compactions to worse consequences up to the non homogeneity of the product obtained.

The reduction of these drawbacks would involve very high costs to carry out the necessary modifications.

For these reasons, with the traditional equipments and production flows, even with the improvements which can be made without causing too high a rise in the price of the product, it would not be possible to obtain powders with an optimum particle size profile, as happens for instance for powdery paints.

In the more specific case of powdery paints, powders are constituted by a part of organic substances and a part of inorganic substances, all of them produced obviously at the solid state.

The organic part comprises in the first place the binder, i.e. a suitable resin, having a molecular weight generally comprised between 500 and 6000 for thermosetting powders and even greater for thermoplastic resins.

The first ones includes epoxy, polyester and acrylic resins; the second ones, especially polyamidic resins.

Together with thermosetting resins, there are also the associated hardeners, which may be, depending on the various systems, phenolic and polyester resins, amines, anhydrides, polyepoxy compositions, such as TGIC, isocyanurates, isocyanates, or isocarboxylic acids.

Besides hardeners, there may be reticulation catalizers (accelerators), which are supplied dispersed in the various types of binders by their producers.

There are also normally extenders, generally polyacrylates, to obtain a better flowability of the layer after its melting and before the hardening, to ensure the homogeneity of the thickness of said layer.

Other optional additives include organic dyes or pigments, benzoin as degasser, thermoplastic polymers as texturizers, dulling waxes, antiscratch waxes.

The inorganic part is constituted by chemically stable natural or artificial inorganic compositions, utilized as inert fillers, which lend the paint body, without altering its colour or dullening it, or as extenders, which can be defined as dullening fillers and which allow therefore to obtain a good hiding power; lastly there are the pigments to obtain the colours; the white ones are utilized as extenders.

As said, both to the utilization and the production purpose, it should be suitable to obtain a particle distribution wherein sizes are shifted towards the medium-low range from 5 to 20 $\mu$ and very little dispersed, with about 80% of the material within a max. range of about $\pm$ 20-30% and negligible percents of particles under 3 $\mu$, which cannot be achieved with the present equipments, because of technical and especially economic reasons.

Therefore, remarkale drawbacks and limitations arise for users which in general hold back the diffusion of powder paints by icreasing their cost, affecting adversely the aspect of finishings and preventing the application of moderate thicknesses.

Said drawbacks include a progressive change in the granule size distribution of the powder during the painting, with a progressive increase in the percent of thinner and coarser particles.

According to the common practice, the powder is sprayed in excess and the powder which does not adhere to pieces is recovered and reintegrated in the fluid bed from which the paint is taken, to send it back to the powder distributors (guns, disks, ...) which electrify the powder and project it towards the pieces by means of compressed air or disk rotation.

The movement of the particles is therefore caused partly by the difference of electric potential between said particles and the items to be painted, and partly by the thrust received, as well as by the motion of air in the cab, caused by the suction of the units for the recovery of the excess powder.

This depends mainly on the fact that the electrization conditions of particles, the quantity of motion supplied to same and the force of the magnetic field, as well as the design of the whole plant are the result of an unavoidable compromise between the requirements imposed by the different sizes of the particles; it is unfavourable to the smallest and greatest ones, which for different reasons settle on the items in a smaller quantity.

Even though amounts of fresh powder corresponding to those that form the paint layer on the items are introduced from time to time into the fluid bed, the drift of the granulometric curve of the powder distributed is unstoppable; even by mixing at intervals the recovered material with a greater quantity of fresh powder one does not eliminate the necessity of rejecting always a part of the recovered material; besides, these operations are not always free from additional costs.

Another drawback associated to the thinnest particles is their greater tendency to agglomerate and to absorb humidity from the air; this causes a limitation of the electrization and a tendency towards the obstruction of the channels for the transport of paint; in its turn, this causes the formation of defects on the painted items and standstills of the plant. This would be much less important if the compromises necessary for the present particle size dispersion could be avoided.

Other difficulties arise from the limited recovery of smaller particles in cyclone systems. The presence of greater particles cause also other drawbacks, such as the difficulty of controlling the thickness of the paint applied, and the ensuing necessity of applying thicknesses greater than necessary, and antiaesthetic results such as the orange peel aspect.

Because of the aforementioned reasons, with the present equipments and production methods, even with the improvements applicable without causing a great increase in prices, it is impossible to obtain powdery paints having an optimum particle size profile.

This field concerns particularly the Applicant which has been utilizing for many years and at present also produces powdery paints, and which is subject to heavy limitations both in discontinuous painting - for which anyhow it shares the common destiny of paint producers - and especially in continuous painting of coils with the special high flexibility paint which has been developed and which is the subject matter of the Italian patent nr. 01237808 and the corresponding patents US 5204168 and EP 433533 by the same Applicant; in fact, for this application it would suffice to realize a film with a max. thickness equal to or lower than 30-40 $\mu$, as it would not be necessary to have recourse to layers having rather great thicknesses, such as for sections or other items having a complex section, in order to cover sufficiently the recesses. One is obliged, because of the characteristics of the present powders, to use greater thicknesses, which increase unduly the cost of the new product, which is in competition with the coils painted with solvent-based paints, which are cheaper and can be applied in thicknesses of 20 $\mu$.

There are further applications of this technology for which the application of thick paint layers, and therefore still smaller particle sizes, would be necessary.

It is clearly impossible to achieve this with products that have particle sizes like the one shown on Table 1 and Fig. 1.

Object of this invention is therefore to provide a process for the production of thin powders having controlled and pre-fixed particle sizes, with a minimum particle size deviation above and under the pre-fixed particle sizes.

A further object of this invention is to provide a process for the production of thin powdery products constituted by mixes of several substances even of a different nature from one another, but intimately mixed and homogeneously distributed in each granule, and also constituted by a matrix formed by an organic solid solution in which inorganic and/or organic compositions outside the solid solution may also be homogeneously dispersed.

A further object of this invention is to provide a process for the production of thin powders which does not involve the utilization of toxic and/or polluting organic solvents.

A further object of this invention is to provide powdery paints having pre-fixed and controlled particle sizes, especially suitable for electrostatic painting.

Still a further object of this invention is to provide a plant suitable to produce thin powders having pre-fixed and controlled particle sizes, with a minimum particle size deviation.

These and still further objects and associated advantages which shall be clear from the following description are achieved by a process for the production of powdery materials having pre-fixed and controlled particle sizes, which process, according to this invention, comprises the following steps:

- melting the material to be pulverized or at least the preponderant part of same or at least the products or substances constituting the preponderant part of the material, if this is constituted by a mix of products or substances, obtaining in this way said material in the state of a fluid or a liquid homogeneous suspension;
- dispersing said liquid material or said homogeneous liquid suspension in a fluid at suitably chosen temperature and pressure, at which said fluid is substantially devoided of any dissolution power relatively to said products or substances, obtaining in this way a solution of droplets in a fluid;
- cooling said droplet dispersion at a cooling temperature lower than the solidification temperature of the product or substance having the lowest melting point of all the products or substances constituting said material, and causing in this way the full solidification of the individual droplets dispersed;
- causing said fluid to evaporate, keeping it at a temperature at least lower than said cooling temperature, obtaining in this way as a residue said powdery material having pre-fixed and controlled particle sizes, with a minimum size deviation and exempt from impurities;
- collecting and recondensing said evaporated fluid and utilizing it to disperse new material or a new liquid suspension.

More particularly, said fluid is constituted by a substance in the state of supercritical fluid, preferably chosen among those having the critical temperature comprised between -20°C and +250°C and the critical pressure comprised between 2 and 80 bar, such as for instance, ethylene, nitrous oxide, ethane, carbon dioxide, trifluoromethane, chlorotrifluoromethane, monofluoromethane, propane, pentane, isopropanole.

The presence in the material also of substances having a high melting point does not jeopardize the result, provided that the volume of the components which remain at the solid state allows them to remain suspended in the liquid phase. The process may be contra-indicated only in the presence of substances having a high vapour tension at the temperature of utilization, which leads them to spread in the supercritical fluid, or which are heat-unstable at temperatures lower or little higher than their melting point, if insulated, and/or than that of the greatest part of the substances present, if we are dealing with a mix.

For ecologic and safety reasons in particular, and also considering a probable small residue of dissolution power also in unfavourable conditions, relatively to some components, carbon dioxide has been taken particularly in consideration, as it does not alterate any ecologic balance, being obtained as a natural product or as a byproduct of various necessary production processes, and being neither toxic nor aggressive, it does not give rise to dangers in case of accidental leaks.

It is now necessary to recall some notions on the supercritical state. As is known, for any substance that is not subject to decomposition during the physical state changes, a complete temperature/pressure diagram comprises, as shown on Fig. 2, two singular points, the first of which, the triple point, is the point of touch between the existence fields of the three states, solid, liquid and gaseous, wherein the sublimation curve of the gaseous state terminates and the fusion curve of the solid state and the evaporation curve of the liquid state start. The end of the latter is the second singular point, the critical point. Table 3, shown farther on, lists some substances with the corresponding values of temperature, pressure and critical density. By further increasing the pressure and/or temperature above the critical ones, no state changes are obtained, but only continuous variations of the characteristics, which are intermediate between those of the liquid and gaseous states; the materials which are in this state are therefore called supercritical fluids.

The properties of supercritical fluids (S.C.F.) change on changing the temperature and/or the pressure; by increasing the temperature they change, nearing the properties of a gas; by increasing the pressure, they near those of a fluid.

In this way one can obtain with the same substance fluids having density and viscosity variable according to the conditions required.

As concerns their utilization as solvents or dispersers, S.C.F.s show, compared to fluids and gases, combinations of more favourable characteristics, arising from their double nature, such as a reduced viscosity, absence of surface tension, high diffusiveness of the dissolved substances, typical of gases, together with the dissolution power, typical of fluids.

Obviously, by variating the temperature and/or the pressure, some properties are exalted and other ones depressed; therefore, it is possible to obtain, with the same substance, fluids having a huge range of different combinations of properties.

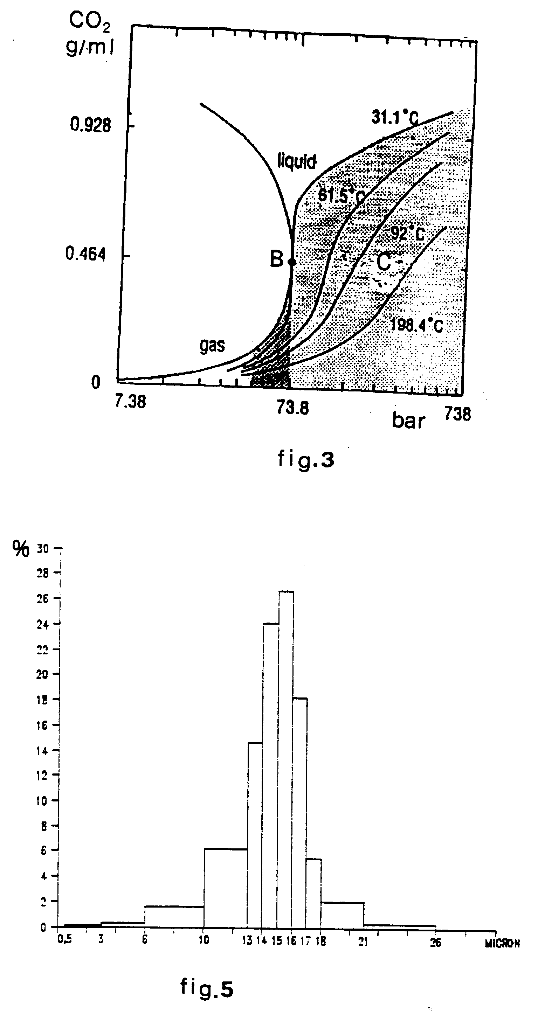

Fig. 3 shows a diagram of the relation between the density of $CO_2$ and the pressure, at different temperatures, in the supercritical zone and in the subcritical ones, where the fluid and the gas still have some resemblances with supercritical fluids.

As the dissolution power of S.C.F.s, as for normal fluids, depends on their chemical characteristics and also on their density, it is possible to obtain the separation of a solute also by acting only on the pressure, with even comparatively small variations; it has also been noticed that the solubility of the substances decreases markedly, together with the density, by increasing the temperature, at least as concerns low vapour tension substances; this effect is opposite to the one found in fluids. Therefore, when one wishes to reduce drastically the dissolution power of a S.C.F, it suffices to adopt pressures that are little higher than the critical one and temperatures not much higher than the critical temperature; Fig. 3 shows that already 30° above the critical temperature, the density decreases drastically.

These conditions of depression of the dissolution power are therefore suitable for the formation of suspensions or dispersions.

A great advantage lies in that one can obtain an immediate separation of the two phases, without leaving any trace of the dispersant fluid which passes in the gaseous stage with very limited pressure variations.

The study of the applications of the S.C.F.s, in spite of the fact that the existence of the supercritical state was known already in the past century, has produced practical applications only from mid-sixties onwards, when the first applications for the chromatographic analyses appeared, but has been given boost especially starting from about ten years ago, and still is a field not totally explored.

Many studies, followed by the respective applications, have concerned besides the chromatographic ones, also the extraction of many substances from a wide range of solid matrices, for both analytical and industrial purposes; between the latter, one should mention, if nothing else for the application scale, the extraction of caffeine from coffee-beans to obtain caffeine-free coffee, to replace th expensive and unsafe extraction with conventional organic solvents.

Hence, most applications exploit S.C.F.s for their dissolution power, especially therefore at high pressures, and the possibility of obtaining the free solute by changing only pressure.

The only application which has some similarities with our problems is the one claimed by several patents of the Battelle Memorial Inst., based on the patent USA 528723 dated 1.9.83, published afterwards also as EP 157827/B1 on 2.12.87. It concerns the production of solid films or powders, even very thin, starting from the solution of a substance in a supercritical fluid; the solution is sprayed in any case on a stiff support, in an environment at the atmospheric or at lower pressure, through a thin channel of pre-fixed dimensions, with a system called "molecular spray", which exploits also the shock waves arising from the rapid inlet of the jet and the "explosive" evaporation of the solvent.

If one changes some conditons, one obtains very thin powders or films even monomolecular.

But both the aims and the technology of this patent are very far from our concepts and needs.

In the field of paints, Union Carbide and Nordson have patented respectively processes and equipments for painting, utilizing, with specific plants, normal formulations of solvent-based paints, in which many organic solvents have been replaced by supercritical state carbon dioxide.

The aim is to reduce markedly the environment pollution by organic solvents, which however, even though in reduced proportions, are sprayed, also by this system, on the items together with the paint, evaporating afterward in the environment. However, all these applications exploit once again the dissolution properties of $CO_2$, exalted, in the case in point, by the presence of co-solvents in small amounts, with processes and problems that are fully different from those of the fabrication of powders.

Studies are also known to have been carried out on the micronization in this powders of individual drugs, also in this case by spraying a solution in supercritical fluids, but no information on industrial applications has been found; this is probably due to the necessity of working at very high pressures, up to 600 bar and more, to obtain an adequate production power.

As concerns the fabrication of complex products, as are powdery paints, we have found no information about any application of S.C.F.s in the technical literature nor from the news that circulate in the concerned milieu.

The process according to this invention has proved to be very advantageous also in the production of powdery paints, which is one of the more complex and difficult fields, because of the dishomogeneity of the constituents.

In fact, in this field the powder particles one wants to obtain must be constituted by a matrix formed by a solid solution or a coherent mix of various organic substances, which may be present even in very different ratios; in such matrix substantial quantities of inorganic susbtances are dispersed and often smaller quantities of organic compositions having a melting point higher than the one of the resin and of most of the other organic additives.

Besides, the various substances present may have chemical and physical characteristics even very different from one another.

According to this invention, one operates, as said, in condition where the various substances are substantially insoluble in the C.S.F.; to obtain this result it is necessary to operate in a field of pressures slightly higher that the critical one, so as to depress the solvent qualities of the supercritical fluid, and at a temperature high enough to melt most of the organic components, without however running the risk of triggering the reticulation of the resin.

Always according to this invention, the hardening substances and the possible reticulation accelerators my be united to the other products which constitute the material to be pulverized, in the case in point the powdery paint, immediately before the phase in which said fluid is caused to evaporate by the dispersion of said droplets; in this way one prevents any risk of untimely reticulation of the resin.

The evaporation of the dispersion fluid is carried out thereafter at a pressure not much lower than the critical pressure of said fluid, for a more economical recovery and recycling of the supercritical fluid.

Further characteristics and advantages of this invention shall appear more clearly from the following detailed description of a preferred non exclusive embodiment, which description makes reference to the attached drawings, which are given only by way of example, wherein:

Fig. 1 shows the typical granulometric curve of the powders obtained by mechanical treatment according to the known art, in particular the typical granulometric curve of powdery paints; the graph shows on the abscissa the intervals of the various fractions in $\mu$, while the ordinate shows the weight percent;

Fig. 2 shows a graph of a typical temperature/pressure state, wherein the temperatures are shown on the abscissa and the pressure logarithm on the ordinate, and the triple point is highlighted by A, the critical point by B, and the zone of the supercritical fluid by C;

Fig. 3 shows the ratio existing between the density of $CO_2$ in g/ml and the pressure in bar, at various temperatures in the supercritic "C" zone and in the subcritic one. The critical point is indicated by B.

Fig. 4 is a block diagram of a discontinuous plant utilized to realize the process subject matter of this invention.

Fig. 5 shows the granulometric curve of a powdery material, in particular a powdery polyester paint, obtained according to the process subject matter of this invention; the graph shows on the abscissa the intervals of the various fractions in $\mu$, while the weight percent is shown on the ordinate.

Fig. 6 shows the granulometric curve of another powdery material, always realized according to the process subject matter of this invention, in particular of phenacetine, while

Fig. 7 shows the granulometric curve of phenacetine, obtained always according to the process subject matter of this invention, but in different working conditions.

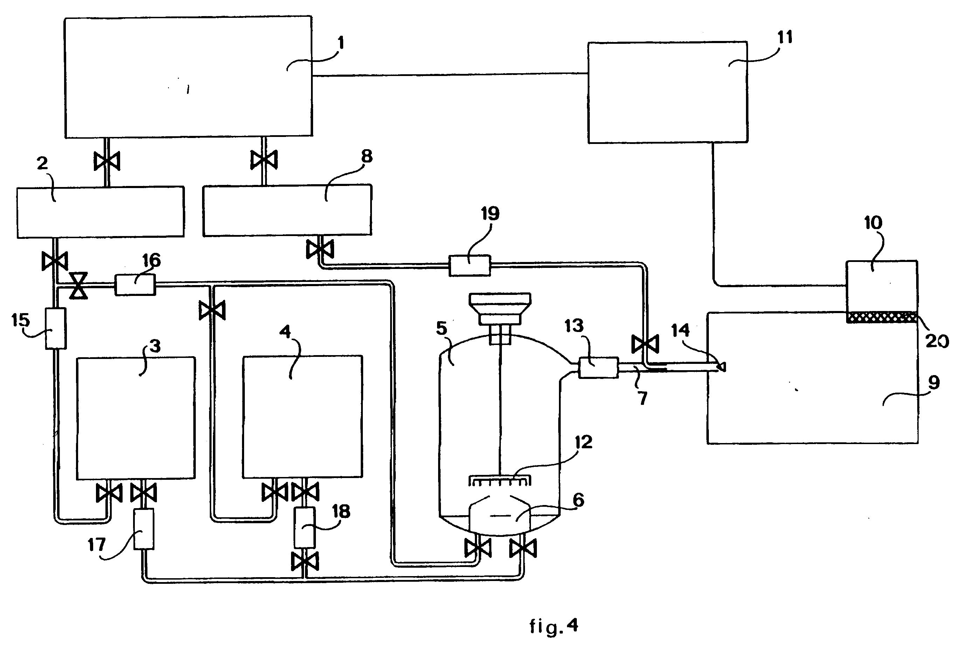

With special reference to Fig. 4, the plant, entirely made from AISI 304 steel, comprises the apparatuses illustrated hereunder:

- 1 is the tank for the liquid S.C.F.;
- 2 is a tank where the S.C.F. is kept at the working temperature, in the quantities necessary for the day;
- 8 is a tank where the S.C.F. is taken from, at low temperature;
- 3 is a constant pressure mixer (adjustable up to 90 bar), with heating jacket walls for temperatures up to 260°C, adjustable by means of a thermostat at $\pm$ 0.5°C. In the latter, the substance(s) are placed at first which constitute the powders to be obtained, except for those which can be in touch with the other ones only for a short time, because of their reactivity towards same. Oncle closed, the container is bleeded and the temperature is brought to the value wished, while the S.C.F. necessary to obtain a rather fluid mix is added. In a continuous production plant, resin and additives may be let in 3, at pre-fixed intervals, drawing them by means of a pump for viscous liquids fron another normal pressure container (not indicated in the diagram), provided with a thermostatically heated mechanical mixer, containing a molten bath of the substances intended for 3; the level is mantained by the automatic addition of new solid materials after each drawing. Obviously, also the corresponding addition of preheated S.C.F. shall be made in 3;

- 4 is a smaller mixer having characteristics analogous to those of 3.

It contains, at the molten state, the substances that cannot remain in touch any longer with those let in 3, besides the S.C.F. necessary for the fluidization.

For this latter too the same considerations hold good as for 3, in relation to a production plant.

If there is one only substance or if no reactive substances are present, the branch containing 4 has no reason for becoming operational and is excluded;

- 5 is the reactor where the formation of the suspension of drops of liquefied material of the wished size is controlled.

A flow reaches it which comes from 3, already mixed during the route with the fluid coming from 4, which is injected in the suitable ratios. Such flow, together with the S.C.F. coming from 2, is let in a premixing chamber having a suitable shape, whose outlet leads the mix just under a suitable disperser 12 which forms a suspension of droplets having the sizes wished. In some cases there may be two or more dispersers at different heights, of a same or different conception and size.

From 5 the suspension goes, through pump 13, to a tube 7 provided with an external cooler, so as to lower the temperature relatively to the preceding working temperature, but keeping it still some degrees above the critical temperature of the S.C.F. utilized, causing the solidification of the drops of material. The tube is tapered, so that its inner diameter decreases in the direction of the motion of the liquid. This is to compensate S.C.F.'s volume reduction.

Alternatively, or also together with the external cooling, cool S.C.F. coming from 8 is let in tube 7.

The temperature and quantity of same may also be so adjusted as to cause the volume increase to compensate for the contraction of the hot suspension. In this case the tube needs not being tapered.

The suspension of solid particles of material is unloaded from the tube into a chamber 9 for the vaporization of the S.C.F. and the collection of the powder, through a valve 14 whose opening is adjusted according to the pressure value in the tube just before the operation, to avoid the untimely vaporization of the S.C.F.

The pressure in the chamber is kept a few bar under the critical one, through the regulation of the gas suction device 10, which gas is filtered through filter 20 and conveyed to the recovery unit 11.

This allows remarkable savings in the recycling of the S.C.F. and makes also untimely gassifications in the pipe which let suspension in 9 more difficult, allowing also a greater and more regular flow than the flow that would be obtained by lower pressures.

In some cases we found it useful to insert, just after the injection point of the fluid coming from 4 in that coming from 3, a length of tube shaped as one or more rings (the detail is not shown on the diagram) to facilitate the complete and homogeneous mixing of the substances coming from the two flows, conveying them in one only continuous phase, thanks to the density difference between the suspended substances and the carrying fluid.

A variant consists in the utilization, instead of disperser 5, of a length of tube provided with one or several dispersor from pipings, preferably preceded by one or more coils of ring-shaped tube.

The equipment has also been utilized, by way of comparison, without the final cooling, lowering the temperature by means of a spray of liquid S.C.F. to allow the solidification of the drops of material.

At the beginning, the product(s) which are to be utilized are let in 3 and possibly in 4; to remove from them as much air as possible, a $10^{-6}$ bar vacuum is created in them; then the S.C.F. is let in at the working pressure, creating again a $10^{-6}$ bar vacuum, then the heating system of 3 and 4 is adjusted to the working temperature and at the same time the S.C.F. is let in at the temperature and pressure wished.

At the same time, the heating is activated in the rest of the plant which is filled with S.C.F. at working pressure and temperature, letting it in just after the valves at the outlet of 3 and 4 to remove the air from it and especially from chamber 9; this part of the S.C.F. is conveyed to a storing container, not represented, to be recycled separately. Now one starts to send the mix from 3 and from 4.

When the plant is standstill, it is possible to let the S.C.F. in the two tanks 3 and 4, causing it to pass through the pipings, after the suspension, to remove the residues of the latter. This is made automatically, as soon as tanks empty out. When a fair amount of S.C.F. has passed, one closes the valves upstream from the nozzle and downstream from 3 and 4, so that all the plant parts comprised between same remain still filled with S.C.F.; if it is not necessary to open the chamber, one closes also the valve upstream from the exhaust fan.

The whole process is controlled by a programmer connected to a computer, not shown on the drawing, which receives the signals coming from the sensors and to which all the actuators for the necessary interventions are connected; the data are recorded for a complete documentation and elaboration of the results.

The main circulation pumps are indicated by 15, 16, 17, 18 and 19. All pipings and pumps are thermostated at working temperature by means of temperature sensors corresponding to the various sections of electric heating bands which are wrapped up around pipings and pumps.

The maintenance of the working pressure is based on a set of pressure sensors located inside the containers and the tubes before and after the pumps and next to the outlet, as well as in 9.

Special apparatuses provide the information on the flow and velocity of the fluid in the critical points.

By means of the plant described above many samplings of powders have been produced, utilizing formulations of paints based either on thermosetting resins or on thermoplastic resins, either comprising or not comprising high melting point additives; also various pure organic compositions have been tested, which do not belong to the field of paints.

With the plant equipped as shown on the diagram, we have obtained, with the insertion in some cases of one or more coils of ring-shaped tube, powders with practically spherical granules with mean sizes comprised between about 5 and 150 $\mu$, with at least the 80% of the weight comprised between $\pm$ 18-26 relatively to the mean size of particles.

By utilizing the plant with the dispersant equipments within the tube, substantially spherical particles have been obtained having the same sizes as in the preceding case, with a slightly greater dispersion, i.e. with 80% of the weight comprised between $\pm$ 23-30% compared to the mean size.

Spraying the suspension without a prior solidification of the suspended drops leds to the formation of particles having a less regular shape, with equal mean sizes but with a greater dispersion, inversely proportional to the pressure in chamber 8. 80% of the weight was distributed, in these conditions, within a range of $\pm$ 30-60% and sometimes even greater, with the rest of the relative weight represented for the most part by smaller particles.

These results have been obtained by utilizing, for the different products to be pulverized and to obtain the various particle sizes, different process parameters, especially as concerns the temperature and the ratio between the quantity of dispersed material and the dispersant means, and also dispersant equipments have been utilized which were different in size as well as in the design; in some cases also the pressure of chamber 9 has been changed.

$CO_2$ has been prevailingly utilized as S.C.F., but tests have been carried out - with analogous results but different parameters - with nitrous oxide, ethylene and chlorotrifluoromethane. The differences between carbon dioxide and nitrous oxide are very limited, which cause them to be practically interchangeable in most cases, while ethylene, because of its nature, has shown to have a residual solvent power that is not negligible compared to several types of organic substances, and has also a lower density which may give rise to problems in the formation of the suspension. Also chlorotrifluoromethane has shown too great a residual dissolution power towards some organic substances other than ethylene.

Concerning the residual dissolution power, which is generally very limited if one works with one only substance or with different substances having similar solubilities, it can be neglected; its only effect is that of increasing the quantity of finer powder, of 1-2 $\mu$ or less, according to the process parameters.

On the contrary, there can be remarkable effects if one works with complex mixes in precise ratios, especially if one or more of them show a certain solubility and if, at the same time, they are present in very small amounts. This is a typical situation one easily finds in powdery paints, whenever organic dyes or pigments are present to obtain a given colour; it happens very often that to remain within the limits of the colour, especially if it is a clear one, the presence of one or more substances in an amount of less than 1% or little more is needed. If, at the process temperature, one of more of them have a vapour tension high enough and are chemically suitable to solve in the S.C.F. utilized, one runs the risk of losing a remarkable part of same in the powder particles having the wished size, and are found instead isolated as very thin particles. If they are irreplaceable, it is necesary to increase their weight in the formulation until one has the necessary quantity in the useful size particles, and bear the charge of a separation by ventilation of the powder obtained; as only particles much thinner than the bulk are to be removed, this operation is not very difficult and its costs can be reabsorbed.

Also tests on the production power have been carried out, obtaining production times which allow industrial plants to reach a powder production power of up to 2000 kg/h.

Obviously, different production powers require the utilization of pipings and a nozzle having a diameter proportional to same.

EXAMPLE 1

Production of a polyester powdery paint, colour ivory RAL 1013 glossy, for sections.

Formulation of the powder:

| PRODUCTS | WEIGHT, GRAMS |
|---|---|
| URALAC P 3500 RESIN (mean mol. weight 5200 acid value 30-36) | 546.00 |
| ARALDIT PT 810 HARDENER (triglycydyl isocyanurate) | 44.70 |
| EXTENDER (2-ethylhexyl acrilate copolymers) | 65.00 |
| ANTISCRATCH WAX (polyethylene chains with a mol. weight between 1000 and 1500) | 2.50 |
| BENZOIN ($\alpha$-hydroxy-benzyl-phenylketone) | 4.00 |
| TITANIUM DIOXIDE | 284.00 |
| BARIUM SULPHATE | 52.18 |
| YELLOW FERRIC OXIDE | 3.12 |
| RED FERRIC OXIDE | 0.05 |
| BLACK FERRIC OXIDE | 0.90 |
| TOTAL | 1002.45 |

The following parameters have been utilized for this formulation:

| | |
|---|---|
| Fluid utilized | carbon dioxide |
| Working T and P | 136°C; 75 bar |
| Substances/fluid ratio in 3 and 4 | 1:1.5 V/V |
| Substance/fluid ratio in 5 | 1:3.4 V/V |
| Temperature after cooling | 38°C |
| Pressure in the collection chamber | 67 bar |
| Equivalent hourly production | 2000 kg/h |
| Mean particle size of powder | 15 $\mu$ |

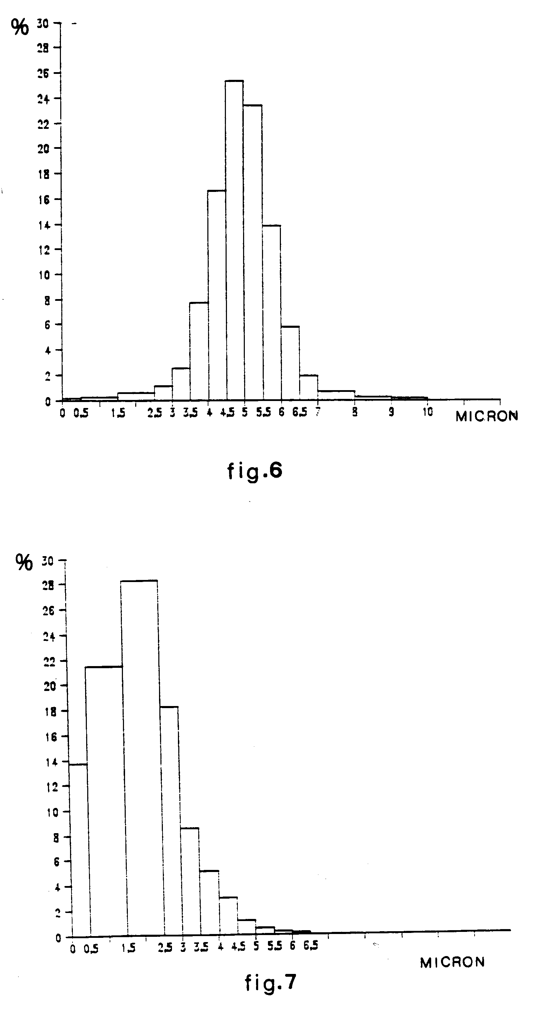

The results of the granulometric measurements on the powder obtained are shown on Table 2 and Fig. 5.

As can be seen, the curve is very different from the typical curve of Fig. 1; about 84% of the powder weight is comprised in an interval of about ± 13% compared to the mean; besides, the fractions above 20 and under 0.5 $\mu$ are almost absent, and those above 21 and under 6 $\mu$ are very limited.

The curve is slightly asymmetrical at it results from the superposition of two effects; the first one is an effect of symmetrical distribution of the sizes of the droplets produced by the disperser, while the second effect, asymmetrical, is due to the impacts of the particles against the walls and among one another, which leads to a certain number of frationings and coalescences, the latter associated to a slight fractioning.

TABLE 2

| GRANULOMETRIC ANALYSIS OF THE POWDER OF EXAMPLE 1 | | |
|---|---|---|
| INTERVALS μ | % WEIGHT ON THE TOTAL | % PROGRESSIVE WEIGHT |
| 0.5 - 3 | 0.1 | 0.1 |
| 3 - 6 | 0.3 | 0.4 |
| 6 - 10 | 1.6 | 2.0 |
| 10 - 13 | 6.2 | 8.2 |
| 13 - 14 | 14.7 | 22.9 |
| 14 - 15 | 24.2 | 47.1 |
| 15 - 16 | 26.7 | 73.8 |
| 16 - 17 | 18.3 | 92.1 |
| 7 - 18 | 5.5 | 97.6 |
| 18 - 21 | 2.1 | 99.7 |
| 21 - 26 | 0.3 | 100.0 |

EXAMPLE 2A

Production of phenacetine [N(4-ethoxyphenyl)acetamide] in thin powder.

This preparation has been carried out to have a comparison with the only case of preparation by evaporation of a solution in S.C.F. for which we know, at least partly, the characteristics of the product obtained.

The information was included in a publication by H.Lot and E.Hemgesberg, of the Saar University, published in the International Journal of Pharmaceutics, Vol. 32 (1986), pages 265-267.

L. and H. have carried out comparative tests on the particles obtained by impact micronization (jet milling) and rapid crystallization due to expansion of the solutions in carbon dioxide (60°C and 600 bar) and trifluoromethane (80°C and 500 bar), obtaining, by evaporation in the normal environment conditions, particles of irregular shape connected by thin filaments.

The specific surface results to be markedly greater for the phenacetine obtained by these methods than for the phenacetine obtained by jet milling; this involves advantages for its wettability and therefore for its dissolution velocity.

Unfortunately the granulometric details are not given but one can obtain some information from the microphotographs obtained with the scanning microscope.

Jet milling forms rounded but not spherical particles, whose sizes are included between about 1 (at least the visible ones, but a thinner powder should be present) and about 12 μ.

The evaluation of the sizes of the products obtained by the solutions in S.C.F. is very difficult, as they have the aspect of a spongy mass; however, it seems that the sizes might be of the same order of magnitudine as those obtained mechanically, the surface increase being probably due to the filaments and the fringed shape of the particles. As we could not find the trifluoromethane at the time of the tests, we have chosen the chlorotrifluoromethane, which has rather similar characteristics (see Table 3).

TABLE 3

| PARAMETERS OF SOME COMPOSITONS AT THEIR CRITICAL POINT | | | |
|---|---|---|---|
| COMPOSITION | CRITICAL TEMPERATURE °C | CRITICAL PRESSURE bar | CRITICAL DENSITY g/cm$^3$ |
| ETHYLENE | 9.3 | 50.4 | 0.218 |
| TRIFLUOROMETHANE | 25.9 | 47.5 | 0.521 |
| CHLOROTRIFLUOROMETHANE | 28.8 | 38.7 | 0.579 |
| CARBON DIOXIDE | 31.1 | 73.8 | 0.464 |
| ETHANE | 32.2 | 48.8 | 0.203 |
| NITROUS OXIDE | 36.5 | 72.7 | 0.450 |
| MONOFLUOROMETHANE | 44.6 | 58.8 | 0.300 |
| PROPANE | 96.8 | 42.6 | 0.217 |
| AMMONIA | 132.5 | 112.8 | 0.235 |
| PENTANE | 196.6 | 33.7 | 0.232 |
| ISOPROPYL ALCOHOL | 235.3 | 47.6 | 0.273 |
| CYCLOHEXANE | 280.0 | 40.5 | 0.273 |
| CYCLOHEXANOL | 356.0 | 38.5 | 0.273 |
| WATER | 374.2 | 220.5 | 0.315 |

The following parameters have been utilized:

| | |
|---|---|
| Fluid utilized | chloro-trifluoromethane |
| Working T and P | 140°C; 41 bar |
| Substance/fluid ration in 3 | 1:1.5 V/V |
| Subsance/fluid ratio in 5 | 1:4.5 V/V |
| Solidification temperature | 32°C |
| Pressure in the collection chamber | about 30 bar |
| Equivalent hourly production | 1000 kg/h |
| Mean size of the suspended drops | 5 µ |

Table 4 and Fig. 6 show the results of the granulometric measurements of the powder obtained.

TABLE 4

| GRANULOMETRIC ANALYSIS - EXAMPLE 2A | | |
|---|---|---|
| INTERVALS $\mu$ | % WEIGHT ON TOTAL | PROGRESSIVE % WEIGHT |
| < 0.5 | 0.1 | 0.1 |
| 0.5 - 1.5 | 0.2 | 0.3 |
| 1.5 - 2.5 | 0.6 | 0.9 |
| 2.5 - 3.0 | 1.1 | 2.0 |
| 3.0 - 3.5 | 2.5 | 4.5 |
| 3.5 - 4.0 | 7.7 | 12.2 |
| 4.0 - 4.5 | 16.6 | 28.8 |
| 4.5 - 5.0 | 25.3 | 54.1 |
| 5.0 - 5.5 | 23.4 | 77.5 |
| 5.5 - 6.0 | 13.8 | 91.3 |
| 6.0 - 6.5 | 5.8 | 97.1 |
| 6.5 - 7.0 | 1.9 | 99.0 |
| 7.0 - 8.0 | 0.7 | 99.7 |
| 8.0 - 9.0 | 0.2 | 99.9 |
| 9.0 - 10.0 | 0.1 | 100.0 |
| > 10 | 0.0 | |

The curve has a dispersal greater than the curve of Example 1, but still good; about 79% of the powder weight is comprised in the interval of about ± 20% compared to the mean size of 4.9 $\mu$; about 92.5% is comprised in the interval of about ± 30%. On the other hand, greater dispersions correspond to the thinner particles.

The powder obtained has a greater homogeneity compared to the powder obtained by jet milling, as the extreme fractions are very reduced, especialy towards the high sizes.

Table 6 shows the results of the permeability tests carried out with the same type of apparatus utilized by the authors of the mentioned study to obtain information on the width of the surface per volume unit typical of the powder.

Under this point of view the powder according to this invention have given results intermediate between those of the jet milling and those of evaporation from $CO_2$, which seems to mean that actually the powder according to this invention is on the average thinner than the one obtained by jet milling.

EXAMPLE 2B

Still further tests have been carried out on phenacetine, to try to obtain a powder more similar to the one obtained by evaporation from solutions in carbon dioxide and in trifluoromethane.

The preparation referred to has been carried out in the same way as the above example, except for the fact that the suspension in chlorotrifluoromethane has not been allowed to solidify in the piping, but has been let in the collection chamber, cooled by evaporation of liquid $CO_2$, whose jet was orientated towards the outlet of the suspension; the mean temperature of the chamber was about 35°C and the pressure was kept at 1 bar, to obtain "explosive" effects of the particles.

The results of the granulometric analysis of the powder obtained, shown on Figs. 6 and 7, stress the great difference between the two systems utilized. While the suspended drops had the same diameter, in this case solid particles have been obtained whose mean size was equal to 2 $\mu$, with an abundant fraction under 0.5 $\mu$ and the disappearance of the fractions above 5 $\mu$.

TABLE 5

| GRANULOMETRIC ANALYSIS - EXAMPLE 2B | | |
|---|---|---|
| INTERVALS μ | % WEIGHT ON TOTAL | PROGRESSIVE % WEIGHT |
| < 0.5 | 13.7 | 13.7 |
| 0.5 - 1.5 | 21.4 | 35.1 |
| 1.5 - 2.5 | 28.2 | 63.3 |
| 2.5 - 3.0 | 18.2 | 81.5 |
| 3.0 - 3.5 | 8.5 | 90.0 |
| 3.5 - 4.0 | 5.1 | 95.1 |
| 4.0 - 4.5 | 3.0 | 98.1 |
| 4.5 - 5.0 | 1.1 | 99.2 |
| 5.0 - 5.5 | 0.5 | 99.7 |
| 5.5 - 6.0 | 0.2 | 99.9 |
| 6.0 - 6.5 | 0.1 | 100.0 |
| > 6.5 | 0.0 | |

All this is due to the sudden variation of pressure and to the impact waves generated by the rapid expansion of the gas.

The drops of phenacetine, before solidifying, almost explode, dividing into smaller fractions, dispersing even very minute particles. The dispersion found was very high: only 76.3% of the powder was comprised in the interval of ± 75% compared to the average. The shape of granules was very different from the substantially sperical one shown by the particles solidified in the tube; they have often the shape of edge-torn disks, or oblong twisted forms or other rather indescribable ones. However, the reticular structures of the powders obtained by Loth and Hemgesberg were not observed.

The permeability tests gave results rather similar to those of phenacetin from solutions in thrifluoromethane, confirming the deviation from regular shapes.

TABLE 6

| AREA VALUES PER VOLUME UNIT FROM PERMEABILITY TESTS CARRIED OUT BY LOTH-HEMGESBERG AND OUR EXAMPLES 2A AND 2B, in $cm^2/cm^3$ | | | | |
|---|---|---|---|---|
| FROM JET MILLING | FROM $CO_2$ | FROM $ClCF_3$ | ES.2A | ES.2B |
| 14.5 | 23.8 | 34.1 | 19.2 | 31 |

## Claims

1. Process for the production of powdery materials having pre-fixed and controlled particle sizes, with a minor size deviation, characterized in that it comprises the following stages:

   - melting the material to be pulverized or at least a preponderant part of same or at least the products or substances constituting the preponderant part of the material when the latter is constituted by a mix of products or substances, obtaining in this way said material in the state of a fluid or a homogeneous liquid suspension;
   - dispersing said liquid material or said homogeneous liquid suspension in a supercritical fluid devoided of any dissolution power for said products and substances, obtaining in this way a dispersion of droplets in a fluid;

- cooling said dispersion of droplets at a cooling temperature lower than the solidification temperature of the product or substance having the lowest melting point among the products or substances constituting said material, and causing in this way the individual droplets to solidify completely in the dispersion;
- causing said fluid to evaporate, keeping it at a temperature lower than said cooling temperature, obtaining in this way as a residue said powdery material having pre-fixed and controlled particle sizes, with a minor size deviation and free from impurities;
- collecting and re-condensing said evaporated fluid and utilizing it to disperse new material or new liquid suspension.

2. Process according to claim 1, characterized in that said fluid is a substance in the state of supercritical fluid chosen among those having the critical temperature comprised in the interval -20°C and +250°C and the critical pressure comprised between 2 and 80 bar, such as for instance ethylene, nitrous oxide, ethane, carbon dioxide, trifluoromethane, chlorotrifluoromethane, monofluoromethane, propane, pentane, isopropanole.

3. Process according to claim 1, characterized in that said powdery materials are powdery paints.

4. Process according to claim 1, characterized in that the substances that may react with other substances constituting the material are united to said other substances or products which too constitute the material, just before the phase of achievement of the controlled dispersion.

5. Powdery material having pre-fixed and controlled particle sizes obtained according the process according to claim 1, characterized in that it is constituted by granules having a substantially spherical shape with variation of deviation of particle sizes lower than $\pm$ 30% relatively to the chosen mean size.

6. Apparatus for the production of powdery material having pre-fixed and controlled grain sizes from a material to be pulverized constituted by a substance or by a mix of component substances, characterized in that it comprises:

- a first tank containing liquid supercritical fluid (S.C.F.);
- a second thermostated tank wherein the S.C.F. is brough to the working temperature:
- a first mixer for mixing at least a first part of said substances with the S.C.F., at a pressure constant and adjustable in the interval from the atmospheric pressure to 90 bar, provided with a heating wall for temperatures from room temperature to 260°C with temperature adjuster at $\pm$ 0,5°C;
- a second mixer, equal to said first mixer, to mix the rest of said substances with the S.C.F.;
- a reactor for the preparation of the dispersion of droplets of material at the liquid state into the fluid at the supercritical state, said reactor being provided with a premixing chamber and a dispersant;
- a tube provided with an external cooler wherein said dispersion coming out from said reactor is brought to a temperature lower than the solidification point of the component substance having the lowest melting point and slightly higher than the critical temperature of the S.C.F., so as to cause the solidification of the dispersed droplets of material;
- a third thermostated tank wherein the S.C.F. coming from said first tank is cooled and then injected in said tube, the temperature and quantity of said S.C.F. being so adjusted as to cause its volume increase to compensate for the contraction of said dispersion;
- a vaporization chamber wherein said dispersion coming from said tube is let in, and wherein the vaporization of the S.C.F. and the collection of the powdery material take place, said chamber being provided with a valve whose opening is adjusted according to the pressure value in said tube, said valve being located between said tube and said vaporization chamber, the pressure in said chamber being kept slightly lower than the critical pressure of the S.C.F. utilized, by suction of said vaporized S.C.F.;
- device for filtering said vaporized S.C.F. and suitable apparatus for recondensing and recycling the S.C.F. to said first tank;
- circulation pumps;
- programmer and associated process computer;
- sensors for recording the temperatures, pressures and flows of the fluids and actuators for the adjustment of temperatures, pressures and flows to the values fixed in the various points of the plants.

7. Apparatus according to claim 7, characterized in that said tube is so tapered that its inner diameter decreases in the direction of the motion of said dispersion, in order to compensate for the reduction in volume of the S.C.F.

8. Apparatus according to claim 7, characterized in that said S.C.F. is constituted by carbon dioxide.

## Patentansprüche

1. Verfahren zur Herstellung von pulverförmigen Stoffen, enthaltend festgesetzte und geregelte Korngrößen, mit einer niedrigeren Abweichung der Größe, dadurch gekennzeichnet, daß es aus den folgenden Schritten besteht:

   - der zu pulverisierende Stoff, oder mindestens ein überwiegendes Teil davon, oder mindestens die Produkte oder Substanzen, die das überwiegende Teil des Stoffes darstellen, wann der letzte aus einer Mischung aus Produkten und Substanzen besteht, werden geschmolzen, dadurch man den genannten Stoff in Form von einem Fluid oder als eine gleichartige flüssige Suspension durchführt;
   - der so erhaltene flüssige Stoff oder die genannte gleichartige flüssige Suspension werden in ein superkritisches Fluid dispergiert, wobei irgendwelches Dipersionsvermögen den Produkten oder Substanzen entzogen wird, dadurch eine Dispersion der Tropfen in ein Fluid durchgeführt wird;
   - die genannte Dispersion der Tropfen wird bei einer niedriger Kühltemperatur gekühlt, als die Erstarrungstemperatur des Produktes oder der Substanz, die den niedrigsten Schmelzpunkt zwischen den diesen Stoff bildenden Produkte oder Substanzen besitzen, so daß die einzelnen Tropfen in der Dispersion völlig erstarrt werden;
   - man läßt das Fluid evaporieren, haltend es bei einer niedrigeren Temperatur, als die obengenannte Kühltemperatur, wobei dieser pulverförmige Stoff als einen Rückstand erhalten wird, welcher festgesetzte und geregelte Korngrößen besitzt, mit einer niedrigeren Abweichung der Größe und befreit von Unvereinigungen;
   - das evaporierte Fluid wird aufgehoben und nochmal verdichtet und angewendet zur Dispergierung des neuen Stoffes oder der neuen flüssigen Suspension.

2. Verfahren nach Anspruch 1, dadurch gekennzeichnet, daß dieses Fluid als eine Substanz in Form von einem superktitischen Fluid vorliegt, ausgewählt aus denen, die eine kritische Temperatur im Intervall zwischen -20° C und + 250° C und einen kritischen Druck zwischen 2 bis 80 bar, wie zum Beispiel, Äthylen, Nitrose, Äthan, Kohlendioxid, Trifluormethan, Chlortrifluormethan, Monofluormethan, Propan, Pentan, Ispopropanol, besitzen.

3. Verfahren nach Anspruch 1, dadurch gekennzeichnet, daß solche pulveförmigen Stoffe pulveformige Lacke sind.

4. Verfahren nach Anspruch 1, dadurch gekennzeichnet, daß die Substanzen, die mit anderen solchen Stoff bildenden Substanzen reagieren können, mit anderen genannten Substanzen, die auch solchen Stoff darstellen, verbunden werden, eben vor der Erreichungsphase der geregelten Dispersion.

5. Pulverformiger Stoff, bestehend aus festgesetzten und geregelten Korngrößen, erhalten gemäß dem Verfahren des Anspruchs 1, dadurch gekennzeichnet daß, es aus Körnern besteht, welche in wesentlich eine kubischer Form besitzen, mit einer um 30% niedrigerer Abweichung der Größe, in Beziehung auf die ausgewählte durchschnittliche Größe.

6. Gebiet zur Herstellung des pulverförmigen Stoffes, welches festgesetzte und geregelte Korngrößen enthält, herauskommend aus einem zu pulverisierenden Stoff, welcher aus einer Substanz oder aus einer Mischung aus Bestandteilen besteht, dadurch gekennzeichnet, daß es aus:

   - einem ersten Tank, enthaltend ein superkritisches Fluid (S.C.F);
   - einem zweiten thermostatischen Tank, worin das S.C.F zur Ansprechtemperatur gebracht ist;
   - einem ersten Rührgefäß, zur Mischung mindestens eines ersten Teiles solcher Substanz mit dem S.C.F., bei einem anhaltenden und im Intervall von Luftdruck bis 90 bar durchsetztlichen Druck, welches mit einem Heizungswand für von Raumtemperatur bis 260°C eingeschlossenen Temperaturen ausgestattet ist, mit einem Temperaturregler von ± 0,5 °C.;
   - einem zweiten Rührgefäß, dem ersten Rührgefäß gleichartig, zur Mischung des Restes solcher Substanz mit dem S.C.F.;
   - einem Reaktor zur Herstellung der Dispersion der Tropfen des Stoffes in flüssiger Form, ins in superkritischer Form vorliegende Fluid, welcher mit einem Mischer, einer Kammer, und einem Dispersionsmittel ausgestattet ist;
   - einer Röhre, ausgestattet mit einem Außenkühler, worin die aus solchem Reaktor herauskommende Dispersion, zu einer niedrigeren Temperatur gebracht wird, als die Erstarrungspunkt der den niedrigsten Schmelzpunkt besitzenden Bestandteilen, und leicht höher als die kritische Temperatur des S.C.F., wobei die evaporierten Tropfen des Stoffes erstarrt werden;
   - einem dritten thermostatischen Tank, worin das aus dem ersten Tank herauskommende S.C.F gekühlt und nachher, in die obengenannte Röhre, eingespritzt wird, wobei die Temperatur und die Größe dieses S.C.F. so eingesetzt werden, daß das Volumen davon zur Ausgleichung der Kontraktion der obegenannten Dispersion erhöht wird;

- einer Verdampfungskammer, worin die aus solcher Röhre herauskommende Dispersion eindringt, und worin das S.C.F. eingedampft und der pulverförmige Stoff aufgehoben werden, welche mit einem Ventil ausgestattet ist, dessen Öffnung gemäß dem Druckventil dieser Röhre eingesetzt ist, welches sich zwischen dieser Röhre und der Verdampfungskammer befindet, und worin der Druck leicht niedriger gehalten wird, als der kritische Druck des angewendeten S.C.F, durchs Saugen des evaporierten S.C.F. ;
- einem Apparat zur Filterung dieses verdunsteten S.C.F., und einem geeigneten Gebiet, zur Kondensation und Rückführung des S.F.C. in den ersten Tank;
- einer Umlaufspumpe;
- einem Programmierer mit einem dazugehörigem Computer zur Kontrolle des Verfahrens;
- Fühler zur Eintragung der Temperaturen, der Drücke, der Flüssen und der Fluide und Trieber zur Einsetzung der Temperaturen, der Drücken, der Flüsse, gemäß den Werten, eingesetzt in verschiedenen Teilen des Plans besteht.

7.  Apparat nach Anspruch 6, dadurch gekennzeichnet, daß die obengenannte Röhre so eine konische Form hat, daß der innere Diameter davon in Direktion der Bewegung der obengenannte Dispersion versinkt, zur Kompensation der Volumenbeschränkung des S.C.F.

8.  Apparat nach Anspruch 7, dadurch gekennzeichnet, daß das S.F.C. aus Kohlendioxid besteht.


**Revendications**

1.  Procédé pour la production de materiaux en poudre ayant un granulométrie determinée et contrôlée avec un écart granulométrique minimum caractérisé en ce qu'il comprend les stades suivants:

    - fondre le matériau à pulvériser ou au moins une partie prépondérente de ce matériau ou au moins les produits ou les substances qui costituent la partie prépondérente du matériau lorsque ce dernier est constitué d'un mélange de produits ou substances, en obtenant ainsi ledit matériau à l'état de liquide ou d'une suspension homogène liquide;
    - disperser ledit matériau liquide ou ladite suspension homogène liquide dans un fluide supercritique qui n'est pas un solvant desdits produits et substances, en obtenant ainsi une dispersion de gouttelettes dans un fluide;
    - refroidir ladite dispersion de gouttelettes à une température de refroidissement inférieure à la température de solidification du produit ou des substances ayant le point de fusion le plus bas parmi les produits ou les substances qui constituent ledit matériau et faisant ainsi solidifier complètement les differéntes gouttelettes dans la dispersion;
    - faire évaporer ledit fluide en le maintenant à une temperature inférieure à ladite température de refroidissement, en obtenant ainsi en tant que résidu ledit matériau en poudre à granulométrie determinée et contrôlée, avec un écart granulométrique minimum et exempt d'impuretés;
    - recueillir et recondenser ledit fluide évaporé et l'utiliser pour disperser un nouveau matériau ou une nouvelle suspension liquide.

2.  Procédé selon la revendication 1 caractérisé en ce que ledit fluide est une substance à l'état de fluide supercritique choisie parmi celles qui ont une température critique comprise dans l'intervalle -20° C et + 250°C et la pression critique comprise entre 2 et 80 bar, telle que par exemple l'éthylène, l'oxyde nitreux, l'éthane, l'anhydride carbonique, le trifluoro méthane, le chlorotrifluorométhane, le monofluorométhane, le propane, le pentane, l'isopropanol.

3.  Procédé selon la revendication 1 caractérisé en ce que lesdits matériaux en poudre sont des vernis en poudre.

4.  Procédé selon la revendication 1 caractérisé en ce que les substances qui peuvent réagir avec d'autres substances qui constituent le matériau sont unies auxdites autres substances ou produits qui constituent aussi le matériau, juste avant le stade d'obtention de la dispersion contrôlée.

5.  Materiau en poudre ayant un granulométrie determinée et contrôlée obtenu suivant le procédé selon la revendication 1 caractérisé en ce qu'il est constituée par de grains en forme substantiellement sphérique avec une variation granulométrique inférieur à +/- 30% par rapport à la dimension moyenne choisie.

6.  Appareil pour la production de materiau en poudre ayant une granulométrie determinée et contrôlée à partir d'un matériau qui doit être pulverisé constitué par une substance ou par un mélange de substances composantes caractérisé en ce qu'il comprend:

- un premier bac contenant un fluide liquide supercritique (F.S.C.);
- un deuxième bac thermostaté ou le F.S.C. est porté à la température d'exercice;
- un premier melangeur pour mélanger au moins une première partie desdites substances avec le F.S.C. à une pression constante et reglable dans l'intervalle compris entre la pression atmospherique et 90 bar, muni d'une paroi chauffante pour des temperatures allant de la temperature ambiente à 260°C avec un reglage à +/- 0,5°C;
- un deuxième mélangeur égal audit premier mélangeur, pour mélanger le reste desdites substances avec le F.S.C.
- un réacteur pour la preparation de la dispersion de gouttelettes du matériau à l'état liquide dans le fluide à l'état supercritique, ledit réacteur étant muni d'une chambre de pré mélangeage et d'un disperseur
- un tube muni d'un refroidisseur extérieur où ladite dispersion sortant dudit reacteur est portée à une température inférieure au point de solidification du composant ayant le point de fusion le plus bas et legèrment plus élevée de la temperature critique du F.S.C. de façon à causer la solidification des gouttelettes dispersées du matériau;
- un troisième bac thermostaté où F.S.C. sortant dudit premier bac est refroidi et ensuite injecté dans ledit tube, la température et la quantité dudit F.S.C. étant reglées de façon à ce que l'augmentation de son volume compense la contraction de ladite dispersion;
- une chambre de vaporisation où ladite dispersion, sortant dudit tube, est introduite et où la vaporisation du F.S.C. et la recolte du matériau en poudre ont lieu, ladite chambre étant munie d'une soupape dont l'ouverture est reglée en fonction de la valeur de la pression dans ledit tube, ladite soupape étant placée entre ledit tube et ladite chambre de vaporisation, la pression dans ladite chambre étant maintenue legèrement au dessous de la pression critique du F.S.C. utilisé, par aspiration dudit F.S.C. vaporisé
- un dispositif pour filtrer ledit F.S.C. vaporisé et un appareil idoine pour recondenser et recycler le F.S.C. dans ledit premier bac;
- des pompes de circulation
- un programmateur et un calculateur de processus relatif
- des senseurs pour enregistrer les temperatures les pressions et les flux des fluides et des actionneurs pour le reglage des temperatures des pressions et des flux aux valeurs fixées aux différents points de l'installation.

7. Appareil selon la revendication 6 caractérisé en ce que ledit tube est effilé de façon à ce que son diamètre intérieur diminue dans la direction du mouvement de ladite dispersion, pour compenser la diminution du volume du F.S.C.

8. Appareil selon la revendication 7 caracterisé en ce que ledit F.S.C. est constitué par l'anhydride carbonique.

fig.1

fig.2

fig.3

fig.5

fig. 4

EP 0 661 091 B1

fig.6

fig.7